# EUROPEAN PATENT APPLICATION

(11) **EP 2 106 792 A1**
(43) Date of publication of application: **07.10.2009**
(21) Application number: 08300170.1
(22) Date of filing: 02.04.2008
(51) Int. Cl.: A61K 31/216, A61K 31/519, A61K 31/517, A61P 13/10

(54) **Use of a combination of udenafil and alfuzosin or oxybutynin for the treatment of overactive bladder**

(71) Applicant: Pelvipharm, 75016 Paris (FR)
(72) Inventor: Giuliano, François, 92210, Saint Cloud (FR); Behr-Roussel, Delphine, 78220, Viroflay (FR)
(74) Representative: Cabinet Plasseraud

(57) **Abstract**

The invention relates to a specific combination of two active agents: udenafil and one of alfuzosil and oxybutynin and its use for the treatment of overactive bladder.

## Description

### FIELD OF THE INVENTION

The present invention relates to the use of a specific combination of udenafil and alfuzosin or oxybutynin for the preparation of a medicament for the treatment of overactive bladder disease.

### BACKGROUND OF THE INVENTION

Overactive bladder is a medical condition defined as urgency (with or without urge incontinence), usually with frequency and nocturia, in the absence of other pathologic or metabolic conditions that might explain these symptoms. Urgency is defined as a sudden and compelling desire to pass urine that is difficult to defer. Nocturia is defined as waking one or more times per night to void urine. Incontinence is not a necessary condition for diagnosis because roughly half of the people with overactive bladder do not have incontinence. Patient quality of life is substantially impacted by this disorder as social, psychological, occupational, domestic, physical, and sexual functioning are all affected.

Overactive bladder can also occur as the result of neurological disease (for example, spinal cord injury, cerebrovascular disease, Parkinsonism or multiple sclerosis) or bladder outlet obstruction. The latter is most common in men with benign prostatic hyperplasia, and is sometimes referred to as lower urinary tract symptoms (LUTS). The following three symptomatic groups are included in overactive bladder: (1) increased urinary frequency and urinary urgency alone, (2) increased urinary frequency, urinary urgency and urinary incontinence, and (3) mixed-type urinary incontinence.

The two functions of the bladder are to store and void urine. The normal bladder fills at a physiological rate dictated by the function of the kidneys. The bladder can accommodate large volumes of urine due to the physical properties of the bladder as well as a neural inhibitory system. The inhibitory mechanism is believed to involve inhibition of parasympathetic activity and, possibly, an increase in sympathetic tone to produce detrusor relaxation and allow filling to occur. During filling, the bladder neck and urethra are contracted, preventing leakage. Voiding, or micturition, is characterized by a relaxation of the outlet neck and the urethra followed by contraction of the detrusor muscle. When micturition is complete, the detrusor muscle relaxes and the bladder neck and urethra contract to seal off the bladder and allow bladder filling. The process of micturition involves neural circuits (afferent and efferent neural pathways and central and peripheral neurotransmitters) in the brain and spinal cord that coordinate the anatomic components of the lower urinary tract.

Anticholinergic/antispasmodic drugs are the first choice for overactive bladder as they have been proven to be the most effective agents in suppressing premature detrusor contractions, enhancing bladder storage, and relieving symptoms. Anticholinergic and antispasmodic agents act by antagonizing cholinergic muscarinic receptors, through which different parasympathetic nerve impulses evoke detrusor contraction. Flavoxate is indicated for the symptomatic relief of cystitis, urethritis, prostatitis, and urethrocystitis/urethrotrigonitis. Darifenacin, oxybutynin, solifenacin, tolterodine, and trospium are indicated for the treatment of overactive bladder with symptoms of urge urinary incontinence, urgency, and urinary frequency.
Of the five known muscarinic subtypes, M3 appears to be the most clinically relevant in the human bladder: contraction of smooth muscle, including muscles in the urinary bladder, is mediated mainly by M3 receptors. Inhibition of the muscarinic receptors in the urinary bladder results in decreased bladder contraction, increased residual urine volume, and decreased detrusor muscle pressure. Oxybutynin, tolterodine, darifenacin, solifenacin, and trospium antagonize the effect of acetylcholine at muscarinic receptors on the detrusor muscle and are know as antimuscarinic agents.

Existing therapies for treating overactive bladder are associated with side effects including constipation, visual-accommodation abnormalities, xerophthalmia (dry eyes) and xerostomia (dry mouth), which are poorly tolerated by some users. Therefore, despite the availability of existing treatments, there is a major unmet and growing need for an effective and acceptable medical treatment for overactive bladder. WO2007/113243 disclosed the use of an inhibitor of cyclic guanosine 3',5'-monophosphate-specific phosphodiesterase type 5 (PDE 5) activity optionally combined to a large variety of others therapeutic agents for treating overactive bladder. The Applicant, while searching for an effective and acceptable medical treatment for overactive bladder, found that a specific combination of two active agents: udenafil and one of alfuzosin and oxybutynin is particularly effective for treating overactive bladder.

### SUMMARY OF THE INVENTION

An object of the present invention is a medicament comprising two active agents: udenafil and one of alfuzosin and oxybutynin or a pharmaceutically acceptable salt, solvate, prodrug or hydrate of anyone of udenafil, alfuzosin and oxybutynin.
Another object of the present invention is a pharmaceutical composition comprising two active agents: udenafil and one of alfuzosin and oxybutynin or a pharmaceutically acceptable salt, solvate, prodrug or hydrate of anyone of udenafil, alfuzosin and oxybutynin, in association with a pharmaceutically acceptable excipient.
In an embodiment of the invention, the medicament or the pharmaceutical composition of the invention is in a unit dosage form.
In an embodiment of the invention, each active agent of the medicament or the pharmaceutical composition of the invention is comprised in a distinct dosage form.
Another object of the invention is the use of udenafil and one of alfuzosin and oxybutynin as active agents for the treatment of overactive bladder.
In one embodiment, udenafil and one of alfuzosin and oxybutynin are formulated in a unit dosage form. In another embodiment, each active agent is formulated in separate dosage forms. Preferably, said dosage form is a solid form.
In one embodiment, udenafil and one of alfuzosin and oxybutynin are to be administrated simultaneously. In another embodiment, udenafil and one of alfuzosin and oxybutynin are to be administrated sequentially.
In another embodiment, udenafil and one of alfuzosin and oxybutynin are to be administrated orally.

### BRIEF DESCRIPTION OF THE DRAWINGS

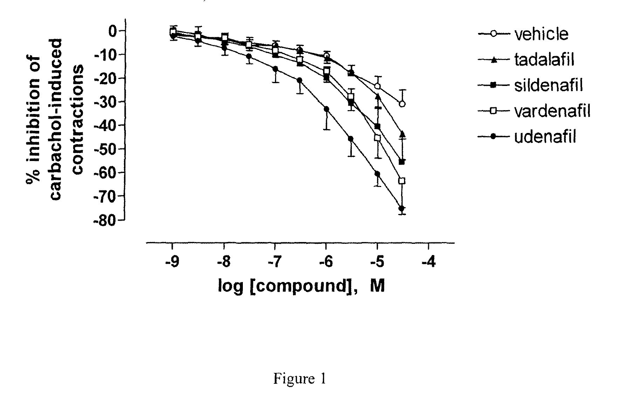

**Figure 1** **:** Effect of PDE5 inhibitors on carbachol precontracted human bladder strips. The data are mean ± SEM of N experiments using bladder samples from N different patients: N= 3 for vehicle, sildenafil, vardenafil, udenafil and N=2 for tadalafil.

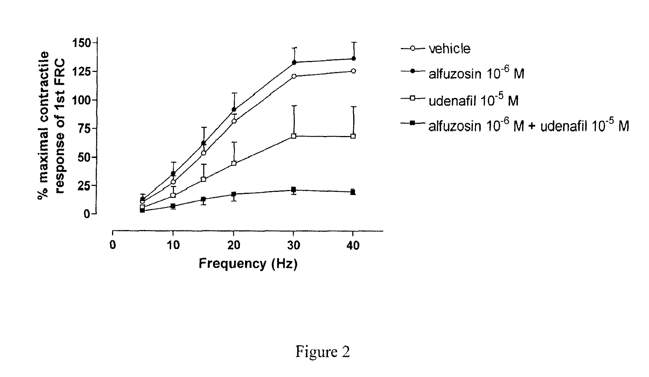

**Figure 2****:** Effect of alfuzosin, udenafil or a combination of both on EFS-induced contractions on human bladder strips. The data are mean ± SEM of N experiments using bladder samples from N different patients: N= 4 for vehicle, alfuzosin, udenafil and N=3 for alfuzosin+udenafil. FRC: frequence response curve

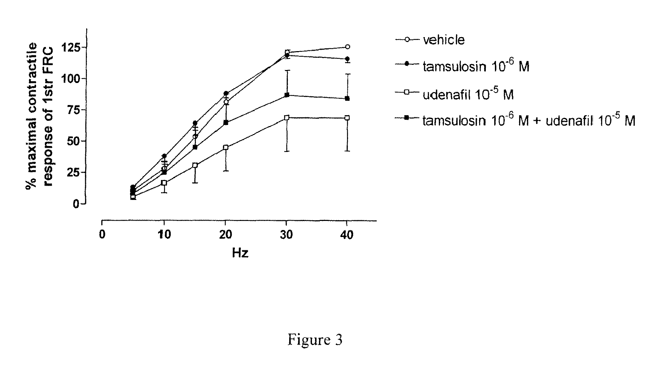

**Figure 3** **:** Effect of tamsulosin, udenafil or a combination of both on EFS-induced contractions on human bladder strips. The data are mean ± SEM of N experiments using bladder samples from N different patients: N= 4 for vehicle, tamsulosin, udenafil and N=3 for tamsulosin+udenafil. FRC: frequence response curve

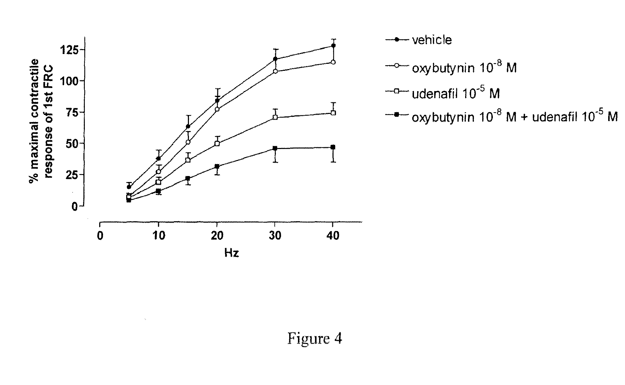

**Figure 4** **:** Effect of oxybutynin, udenafil or a combination of both on EFS-induced contractions on human bladder strips. The data are mean ± SEM of N experiments using bladder samples from N different patients: N= 6 for vehicle and udenafil; N=4 for oxybutynin and oxybutynin+udenafil. FRC: frequence response curve

### DETAILED DESCRIPTION OF THE INVENTION

The Applicant while searching for an effective and acceptable medical treatment for overactive bladder, found that a specific combination of two active agents: udenafil and one of alfuzosin and oxybutynin is particularly effective for treating overactive bladder. It is a feature of the invention that the combination of active agents used in the invention is "synergistic", meaning that the therapeutic effect of co-administering udenafil and one of alfuzosin or oxybutynin is greater than additive.

An object of the present invention is a medicament comprising two active agents: udenafil and one of alfuzosin and oxybutynin or a pharmaceutically acceptable salt, solvate, prodrug or hydrate of anyone of udenafil, alfyzosin and ocybutynin. Said medicament is intended for the treatment of overactive bladder.

Udenafil has the following formula: 5-[2- propyloxy-5-(1-methyl-2-pyrrolidinylethylamidosulfonyl)phenyl]-methyl-3-propyl-1,6- dihydro-7H-pyrazolo(4,3 - d)pyrimidine-7-one.
Alfuzosin is chemically known as (R,S)-N-[3-[(4-amino-6,7-dimethoxy-2-quinazolinyl)methylamino]propyl]tetrahydro-2-furancarboxamide hydrochloride Oxybutynin is chemically known as 4-Diethylaminobut- 2-ynyl2- cyclohexyl-2- hydroxy-2-phenyl-ethanoate.

The invention encompasses all active forms of udenafil, alfuzosin and oxybutynin, including the free form thereof (free acid or base form) and also all pharmaceutically acceptable salts, prodrugs, polymorphs, hydrates, solvates, and stereoisomers. The term "pharmaceutically acceptable salts" refers to any pharmaceutically acceptable salt, which upon administration to the recipient is capable of providing (directly or indirectly) the compounds of the invention.
For instance, pharmaceutically acceptable salts of compounds used in the invention are synthesized from the parent compound which contains a basic or acidic moiety by conventional chemical methods. Generally, such salts are, for example, prepared by reacting the free acid or base forms of udenafil with a stochiometric amount of the appropriate base or acid in water or in an organic solvent or in a mixture of the two. Generally, non aqueous media like ether, ethyl acetate, ethanol, isopropanol or acetonitrile are preferred. Examples of the acid addition salts include mineral acid addition salts such as, for example, hydrochloride, hydrobromide, hyrdroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts. The preparation of salts and derivatives can be carried out by methods known in the art. The term "solvate" as used herein means compounds or a pharmaceutically acceptable salt of the compounds, wherein molecules of a suitable solvent are incorporated in the crystal lattice. A suitable solvent is physiologically tolerable at the dosage administered. Examples of suitable solvents are ethanol, water and the like. When water is the solvent, the molecule is referred to as a "hydrate".
The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Prodrug design is discussed generally in Hardma et al. (Eds.), Goodman and Gilman's The Pharmacological Basis of Therapeutics, 9th ed., pp. 11-16 (1996). To illustrate, prodrugs can be converted into a pharmacologically active form through hydrolysis of, for example, an ester or amide linkage, thereby introducing or exposing a functional group on the resultant product. The prodrugs can be designed to react with an endogenous compound to form a water-soluble conjugate that further enhances the pharmacological properties of the compound, for example, increased circulatory half-life. Alternatively, prodrugs can be designed to undergo covalent modification on a functional group with, for example, glucuronic acid, sulfate, glutathione, amino acids, or acetate. The resulting conjugate can be inactivated and excreted in the urine, or rendered more potent than the parent compound. High molecular weight conjugates also can be excreted into the bile, subjected to enzymatic cleavage, and released back into the circulation, thereby effectively increasing the biological half-life of the originally administered compound. Particularly favoured derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species. The compounds used in the present invention may be in crystalline form either as free compounds or as solvates (e.g. hydrates).
The compounds of the invention or their salts or solvates used in the invention are preferably in pharmaceutically acceptable or substantially pure form. By pharmaceutically acceptable form is meant, "inter alia", having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compounds of the invention, or of its salts, solvates or prodrugs.

Another object of the invention is also a pharmaceutical composition comprising two active agents: udenafil and one of alfuzosin and oxybutynin or a pharmaceutically acceptable salt, solvate, prodrug or hydrate of anyone of udenafil, alfyzosin and ocybutynin, in association with a pharmaceutically acceptable excipient. Said pharmaceutical composition is intended for the treatment of overactive bladder.

By "pharmaceutically acceptable" it is meant the carrier, diluent or excipient must be compatible with the other ingredients of the formulation and not deleterious to the recipient thereof. In general, pharmaceutical compositions comprise effective amounts of the active compound together with pharmaceutically acceptable diluents, preservatives, solubilizers, emulsifiers, adjuvants and/or carriers. Such compositions include diluents of various buffer content (e.g., Tris-HCl, acetate, phosphate), pH and ionic strength; additives such as detergents and solubilizing agents (e.g., Tween 80, Polysorbate 80), anti-oxidants (e.g., ascorbic acid, sodium metabisulfite), preservatives (e.g., Thimersol, benzyl alcohol) and bulking substances (e.g., lactose, mannitol); incorporation of the material into particulate preparations of polymeric compounds such as polylactic acid, polyglycolic acid, etc. or into liposomes. Hyaluronic acid may also be used, and this may have the effect of promoting sustained duration in the circulation. The pharmaceutical compositions optionally may include still other pharmaceutically acceptable liquid, semisolid, or solid diluents that serve as pharmaceutical vehicles, excipients, or media, including but are not limited to, polyoxyethylene sorbitan monolaurate, magnesium stearate, methyl- and propylhydroxybenzoate, starches, sucrose, dextrose, gum acacia, calcium phosphate, mineral oil, cocoa butter, and oil of theobroma. Such compositions may influence the physical state, stability, rate of in vivo release, and rate of in vivo clearance of the inhibitors.

The invention described above comprises a pharmaceutical composition or a medicament and the use thereof, in which an individual dose contains two active agents: udenafil and one of alfuzosin and oxybutynin. In the alternative, the active agents may be administered in separate dosage forms, at the same time or one after the other. For purposes of this application, these alternatives are described as concomitant administration.

Medicament or pharmaceutical composition intended for oral use may be prepared according to any method known in the art for the manufacture of medicament or pharmaceutical composition and such composition may contain one or more agents selected from the group consisting of sweetening agents, flavoring agents, coloring agents and preserving agents in order to provide pharmaceutically elegant and palatable preparations. Tablets contain the active agents in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. Compositions for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin, or olive oil. Aqueous suspensions contain the active materials in admixture with excipients suitable for the manufacture of aqueous suspensions. Oily suspensions may be formulated by suspending the active ingredient in suitable oil. Oil-in-water emulsions may also be employed. Dispersible powders and granules suitable for preparation of an aqueous suspension by the addition of water provide the active ingredient in admixture with a dispersing or wetting agent, suspending agent and one or more preservatives.
Medicament or pharmaceutical composition of the invention may be in the form of a sterile injectable aqueous or oleagenous suspension. The active agents of the invention may also be administered in the form of suppositories for rectal administration. For topical use, creams, ointments, jellies, solutions or suspensions, etc., containing the compounds of the present invention may be employed. The compounds of the present invention may also be formulated for administered by inhalation. The compounds of the present invention may also be administered by a transdermal patch by methods known in the art.

The pharmaceutical composition or medicament of the invention may be presented in unit dosage form and may be prepared by any of the methods well known in the art of pharmacy. The term "unit dosage form" is taken to mean a single dose wherein all active and inactive ingredients are combined in a suitable system, such that the patient or person administering the drug to the subject can open a single container or package with the entire dose contained therein, and does not have to mix any components together from two or more containers or packages. Typical examples of unit dosage forms are tablets or capsules for oral administration, single dose vials for injection, or suppositories for rectal administration. This list of unit dosage forms is not intended to be limiting in any way, but merely to represent typical examples in the pharmacy arts of unit dosage forms. The pharmaceutical composition or medicament of the invention may also be presented as a kit, whereby two or more components, which may be active or inactive ingredients, carriers, diluents, and the like, are provided with instructions for preparation of the actual dosage form by the subject or person administering the drug to the subject. Such kits may be provided with all necessary materials and ingredients contained therein, or they may contain instructions for using or making materials or components that must be obtained independently by the patient or person administering the drug to the patient.

In one embodiment of the invention, the medicament or pharmaceutical composition as described here above is in a unit form.

In another embodiment of the invention, each active agent of the medicament or pharmaceutical composition as described here above is separate dosage forms.

Another object of the invention is the use of udenafil and one of alfuzosin and oxybutynin as active agents for the treatment of overactive bladder. Another object of the present invention is a combination therapy for treating overactive bladder wherein one of the active agents is udenafil or a pharmaceutically pharmaceutically acceptable salt, solvate, prodrug or hydrate thereof, and another is one of alfuzosin and oxybutynin or pharmaceutically acceptable salt, solvate, prodrug or hydrate thereof.
Another object of the invention is a method for treating overactive bladder in a subject in need thereof, said method comprising the co-administration of a therapeutically effective amount of two active ingredients: udenafil and one of alfuzosin and oxybutynin or a pharmaceutically acceptable salt, solvate, prodrug or hydrate of udenafil, alfuzosin and oxybutynin.
The terms "administration of or "administering" an active agent should be understood to mean providing an active agent of the invention to the subject in need of treatment in a form that can be introduced into that individual's body in a therapeutically useful form and therapeutically effective amount, including, but not limited to: oral dosage forms, such as tablets, capsules, syrups, suspensions, and the like; injectable dosage forms, such as IV, BvI, or DP, and the like; transdermal dosage forms, including creams, jellies, powders, or patches; buccal dosage forms; inhalation powders, sprays, suspensions, and the like; and rectal suppositories.
The term "therapeutically effective amount" refers to a sufficient quantity of the active agents of the present invention, in a suitable composition, and in a suitable dosage form to treat or prevent the symptoms, progression, or onset of overactive bladder. The therapeutically effective amount will vary depending on the inhibitor, the state of the subject's overactive bladder or its severity, and the age, weight, etc., of the subject to be treated. A therapeutically effective amount can vary, depending on any of a number of factors, including, e.g., the specific inhibitor, the route of administration, the condition of the subject, as well as other factors understood by those in the art.

The term "treatment" or "treating" generally refers to an intervention in an attempt to alter the natural course of the subject being treated, and may be performed either for prophylaxis or during the course of clinical pathology. Desirable effects include, but are not limited to, preventing occurrence or recurrence of disease, alleviating symptoms, suppressing, diminishing or inhibiting any direct or indirect pathological consequences of the disease, ameliorating or palliating the disease state, and causing remission or improved prognosis.

In one embodiment of the invention, the two active agents: udenafil and one of alfuzosin and oxybutynin are formulated in a unit dosage form, for the treatment of overactive bladder.
In another embodiment of the invention, each active agent is formulated in a separate dosage form, for the treatment and of overactive bladder.

In a preferred embodiment of the invention, said dosage form may be a solid form.
Solid dosage forms include tablets, capsules, pills, troches or lozenges, cachets or pellets. Alternatively, proteinoid encapsulation or liposomal encapsulation may be used.

In a preferred embodiment of the invention, the two active agents: udenafil and one of alfuzosin and oxybutynin are to be administrated orally. In one embodiment, said active agents are in an oral solid form. In a particular embodiment, they may be in a fast-dissolving form or in a controlled-release form. Controlled-release forms encompass formulations comprising the composition incorporated into an inert matrix which permits release by either diffusion or leaching mechanisms e.g., gums. Slowly degenerating matrices, e.g. alginates, polysaccharides, may also be incorporated into the formulation.

In general, the formulation will include a preparation of the invention and inert ingredients which allow for protection against the stomach environment, and release of the biologically active material in the intestine.
If necessary, the compounds used in the invention may be chemically modified so that oral delivery is efficacious. Generally, the chemical modification contemplated is the attachment of at least one moiety to the compound molecule itself, where said moiety permits (a) inhibition of proteolysis; and (b) uptake into the blood stream from the stomach or intestine. Also desired is the increase in overall stability of the compound and increase in circulation time in the body. Examples of such moieties include polyethylene glycol, copolymers of ethylene glycol and propylene glycol, carboxymethyl cellulose, dextran, polyvinyl alcohol, polyvinyl pyrrolidone and polyproline. Other polymers that could be used are poly-1,3-dioxolane and poly-1,3,6-tioxocane.
Compositions can be included in formulation as fine multiparticulates in the form of granules or pellets of particle size about, for example, one mm. The formulation of the material for capsule administration can also be as a powder, lightly compressed plugs or even as tablets. Compositions are optionally prepared by compression.
Compositions including disintegrants are further contemplated in solid dosage form compositions. Materials used as disintegrants include, but are not limited to, starch, sodium starch glycolate, Amberlite, sodium carboxymethylcellulose, ultramylopectin, sodium alginate, gelatin, orange peel, acid carboxymethyl cellulose, natural sponge and bentonite. Another form of disintegrant is an insoluble cationic exchange resin. Powdered gums may also be used as disintegrants and as binders, and these can include powdered gums such as agar, Karaya or tragacanth. Alginic acid and its sodium salt are also useful as disintegrants. Pharmaceutical compositions including binders are further contemplated to hold the therapeutic agent together to form a hard tablet and exemplary binders include materials from natural products such as acacia, tragacanth, starch, and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic. An antifrictional agent in a pharmaceutical composition is further contemplated to prevent sticking during the formulation process. Lubricants include, but are not limited to, stearic acid, including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.
Glidants that might improve the flow properties of a pharmaceutical composition during formulation and to aid rearrangement during compression are also provided. Exemplary glidants include starch, talc, pyrogenic silica and hydrated silicoaluminate. To aid dissolution of a composition into the aqueous environment, incorporation of a surfactant as a wetting agent is contemplated. Exemplary surfactants include anionic detergents such as sodium lauryl sulfate, dioctyl sodium sulfo succinate, and dioctyl sodium sulfonate. Cationic detergents are contemplated, including for example and without limitation, benzalkonium chloride or benzethonium chloride. Compositions using as surfactants lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose are also contemplated. Compositions comprising these surfactants, either alone or as a mixture in different ratios, are therefore further provided. Optionally, additives are included in a pharmaceutical composition to enhance uptake of the compound, such additives including, for example and without limitation, fatty acids oleic acid, linoleic acid and linolenic acid.

In a particular embodiment, controlled release formulations are also provided. A preparation is incorporated into an inert matrix which permits release by either diffusion or leaching mechanisms e.g., gums. Slowly degenerating matrices, e.g., alginates, polysaccharides, may also be incorporated into the formulation. Another form of a controlled release is by a method based on the Oros therapeutic system (Alza Corp.), i.e., the drug is enclosed in a semi-permeable membrane which allows water to enter and push drug out through a single small opening due to osmotic effects. Some enteric coatings also have a delayed release effect.
Other coatings may be used in compositions disclosed herein, including for example, a variety of sugars which could be applied in a coating pan. The compositions also include a film coated tablet and the materials used in this instance are divided into two groups. The first includes the non-enteric materials, such as and without limitation methyl cellulose, ethyl cellulose, hydroxyethyl cellulose, methylhydroxy- ethyl cellulose, hydroxypropyl cellulose, hydroxypropyl-methyl cellulose, sodium carboxy- methyl cellulose, providone and the polyethylene glycols. The second group consists of the enteric materials that are commonly esters of phthalic acid. A mix of materials is also contemplated to provide the optimum film coating. Film coating may be carried out in a pan coater or in a fluidized bed or by compression coating.

Pharmaceutically acceptable carriers include carbohydrates such as trehalose, mannitol, xylitol, sucrose, lactose, and sorbitol. Other ingredients for use in formulations may include DPPC, DOPE, DSPC and DOPC. Natural or synthetic surfactants may be used. PEG may be used (even apart from its use in derivatizing a compound). Dextrans, such as cyclodextran, may be used. Cyclodextrins may be used. Bile salts and other related enhancers may be used. Cellulose and cellulose derivatives may be used. Amino acids may be used, such as use in a buffer formulation. The use of liposomes, microcapsules or microspheres, inclusion complexes, or other types of carriers is also contemplated.

It will be appreciated that when using any combination described herein, the active agents of the invention will be administered to a patient, within a reasonable period of time. In one embodiment of the invention, udenafil and one of alfuzosin and oxybutynin may be in the same pharmaceutically acceptable carrier and therefore administered simultaneously. In another embodiment of the invention, udenafil and one of alfuzosin and oxybutynin may be in separate pharmaceutical carriers such as conventional oral dosage forms which are taken simultaneously. The term "combination" also refers to the case where the compounds are provided in separate dosage forms and are administered sequentially. In another embodiment of the invention, udenafil and one of alfuzosin and oxybutynin may be in separate pharmaceutical carriers such as conventional oral dosage forms which are taken sequentially, within a reasonable period of time.

Therefore, by way of example, one active component may be administered as a tablet and then, within a reasonable period of time, the second active component may be administered either as an oral dosage form such as a tablet or a fast-dissolving oral dosage form. By a "fast dissolving oral formulation" is meant, an oral delivery form which when placed on the tongue of a patient, dissolves within about 10 seconds.
By "reasonable period of time" is meant a time period that is not in excess of about 1 hour. That is, for example, if the first active component is provided as a tablet, then within one hour, the second active component should be administered, either in the same type of dosage form, or another dosage form which provides effective delivery of the medicament.

The active agents of this invention may be administered to subjects (humans and animals, including companion animals, such as dogs, cats and horses) in need of such treatment in dosages that will provide optimal pharmaceutical efficacy. It will be appreciated that the dose required for use in any particular application will vary from subject to subject, not only with the particular active agent or composition selected, but also with the route of administration, the nature of the condition being treated, the age and condition of the patient, concurrent medication or special diets then being followed by the patient, and other factors which those skilled in the art will recognize, with the appropriate dosage ultimately being at the discretion of the attendant physician.

A suitable dosage level of the active agents of the invention is about:
- udenafil : 50 mg to 200 mg per subject per day,
- alfuzosin: 7.5 mg to 10 mg per subject per day,
- oxybutynin: 5 mg to 30 mg per subject per day.

### EXAMPLES

The following examples are provided to illustrate the invention, but are not intended to limit the scope thereof.

### Material and Methods

### Human bladder strips

The experiments, collection and use of any tissue or other samples were carried out in accordance with the Research Plan, all relevant laws, regulations and codes of practice, including having obtaining informed consent of patients in writing.

Bladders were obtained from donors undergoing cystectomy for infiltrating bladder cancer with no known bladder dysfunction according to their medical chart. In accordance with French legislation, human tissue samples were obtained with patient informed consent and after hepatitis and HIV serologies determination.

After surgical procedure, bladder samples were immediately transported from the operating room to the pathologist facilities where a normal piece of the bladder dome, i.e. with no macroscopic tumoral tissue, was selected for experiments by the pathologist. This piece was stored at 4°C in Krebs-HEPES buffer (with the following millimolar composition : NaCl 118.0; KCl 4.7; MgSO4 1.2; KH2PO4 1.2; CaC12 2.5; NaHCO3 4.2; glucose 11.1; HEPES 20.8; pH 7.4) containing penicillin (100 UI/ml) and streptomycin (0.1 mg/ml) for optimal conservation until use (within a maximum of 24 hours).

Then, detrusor strips (8 mm long x 4 mm wide on average) were prepared by removing serosal and mucosal layers. Strips were then mounted isometrically at a resting tension of 500 mg in 5 ml organ baths filled with Krebs-HEPES buffer maintained at 37°C and continuously bubbled with 95%O₂-5%CO₂.

The strips were connected to force transducers (Pioden Controls Ltd, UK) and following amplification, the tension changes were recorded via MacLab™/8 using Chart™ 5 software (AD Instruments Ltd). Following 60 min of equilibration, time during which the buffer solution is changed every 15 min, a priming period is achieved and strips were pre-contracted with KCl (100 mM, 10 min).

### Drugs and chemicals

PDE5 inhibitors were purchased from Alsachim SAS (Strasbourg, France). Alfuzosin and tamsulozin were purchased from Sequoia Research Products (Oxford, UK). Other drugs and chemicals were purchased from Sigma (Saint Quentin Fallavier, France). Initial dilution of 10⁻² M of PDE5 inhibitors was performed in 100 % DMSO. The next dilution at 3.10⁻³ M was also performed in 100% DMSO and the following successive serial dilutions were then in distilled water.
Solubilization of alfuzosin, tamsulozin or oxybutynin was performed in distilled water.

### Data analysis

For the evaluation of the effect of drugs to inhibit carbachol-induced contractions, relaxations in response to increasing and cumulative concentrations of PDE5 inhibitors or oxybutynin or corresponding concentrations of vehicle are expressed as the percentage of inhibition of the contractile response to carbachol.
For the experiments with electrical field stimulation, values are expressed in percentage of the maximal contractile response obtained during the first frequency response curve.

### Experiment 1: comparison of the effect of four PDE-5 inhibitors on carbachol-induced precontracted human detrusor strips.

Strips were primed with carbachol (3.10⁻⁶ M, 10 min), washed repeatedly, pre-contracted with carbachol (10⁻⁶ M) and allowed to re-equilibrate until a stable response is obtained (20-30min).
Then, PDE5 inhibitors (tadalafil, sildenafil, vardenafil and udenafil) or vehicle were added in a cumulative fashion every 5 min at concentrations ranging from 10⁻⁹ to 3.10⁻⁵ M in semi-log increments.
Results are shown figure 1. Udenafil exerts a greater inhibitory effect than the other PDE-5 inhibitors tested to relax carbachol-induced contractions of human bladder strips.

### Experiment 2: Analysis of a combination udenafil/alfuzosin on EFS-induced contractions of human bladder strips

Electrical field stimulations (EFS) are applied to the strips via two platinum electrodes located on either side of the strips and connected to a stimulator (Bionic System Nozay, France). The detrusor strips are primed by applying electrical field stimulation (EFS, 30 Hz, 0.5 ms pulse duration, 5 sec train duration at 300 mA). Stimulations are repeated until stable responses are obtained. A response is considered stable when it is not different from more than 10% of the previous response. After washings, frequency-response curves are constructed: increasing frequencies of electrical stimulation (5, 10, 15, 20, 30, 40 Hz) are applied every 2 minutes. At the completion of the first frequency-response curve, bladder strips are washed, and the strips are incubated with alfuzosin 10⁻⁶M or udenafil 10⁻⁵M or alfuzosin 10⁻⁶M+udenafil 10⁻⁵M or vehicle. Then, a second frequency-response curve is generated with the same EFS parameters than before.

Results are shown figure 2. The specific combination udenafil and alfuzosin exerts a synergistic inhibitory effect on EFS-induced contractions of human bladder strips compared to each compound alone.

### Experiment 3: Analysis of a combination udenafil/tamsulosin on EFS-induced contractions of human bladder strips

Electrical field stimulations (EFS) are applied to the strips via two platinum electrodes located on either side of the strips and connected to a stimulator (Bionic System Nozay, France). The detrusor strips are primed by applying electrical field stimulation (EFS, 30 Hz, 0.5 ms pulse duration, 5 sec train duration at 300 mA). Stimulations are repeated until stable responses are obtained. A response is considered stable when it is not different from more than 10% of the previous response. After washings, frequency-response curves are constructed: increasing frequencies of electrical stimulation (5, 10, 15, 20, 30, 40 Hz) are applied every 2 minutes. At the completion of the first frequency-response curve, bladder strips are washed, and the strips are incubated with tamsulosin 10⁻⁶M or udenafil 10⁻⁵M or tamsulosin 10⁻⁶M+udenafil 10⁻⁵M or vehicle. Then, a second frequency-response curve is generated with the same EFS parameters than before. Results are shown figure 3. The combination udenafil and tamsulosin does not exert a synergistic inhibitory effect on EFS-induced contractions of human bladder strips compared to each compound alone.

### Experiment 4: Analysis of a combination udenafil/oxybutynin on EFS-induced contractions of human bladder strips

Electrical field stimulations (EFS) are applied to the strips via two platinum electrodes located on either side of the strips and connected to a stimulator (Bionic System Nozay, France). The detrusor strips are primed by applying electrical field stimulation (EFS, 30 Hz, 0.5 ms pulse duration, 5 sec train duration at 300 mA). Stimulations are repeated until stable responses are obtained. A response is considered stable when it is not different from more than 10% of the previous response. After washings, frequency-response curves are constructed: increasing frequencies of electrical stimulation (5, 10, 15, 20, 30, 40 Hz) are applied every 2 minutes. At the completion of the first frequency-response curve, bladder strips are washed, and the strips are incubated with oxybutynin 10⁻⁸M or udenafil 10⁻⁵M or oxybutynin 10⁻⁸M+udenafil 10⁻⁵M or vehicle. Then, a second frequency-response curve is generated with the same EFS parameters than before.
Results are shown figure 4. The specific combination udenafil and oxybutynin exerts a synergistic inhibitory effect on EFS-induced contractions of human bladder strips compared to each compound alone.

## Claims

1. Medicament comprising two active agents: udenafil and one of alfuzosin and oxybutynin or a pharmaceutically acceptable salt, solvate, prodrug or hydrate of anyone of udenafil, alfuzosin and oxybutynin.

2. Pharmaceutical composition comprising two active agents: udenafil and one of alfuzosin and oxybutynin or a pharmaceutically acceptable salt, solvate, prodrug or hydrate of anyone of udenafil, alfuzosin and oxybutynin, in association with a pharmaceutically acceptable excipient.

3. Medicament according to claim **1** or pharmaceutical composition according to claim **2,** being in a unit dosage form.

4. Medicament according to claim **1** or pharmaceutical composition according to claim **2,** wherein each active agent is comprised in a distinct dosage form.

5. The use of udenafil and one of alfuzosin and oxybutynin as active agents for the treatment of overactive bladder.

6. The use according to claim **5,** wherein udenafil and one of alfuzosin and oxybutynin are formulated in a unit dosage form.

7. The use according to claim **5,** wherein each active agent is formulated in separate dosage forms.

8. The use according to anyone of claims **5** to **7,** wherein said dosage form is a solid form.

9. The use according to claim **7,** wherein udenafil and one of alfuzosin and oxybutynin are to be administrated simultaneously.

10. The use according to claim **7,** wherein udenafil and one of alfuzosin and oxybutynin are to be administrated sequentially.

11. The use according to anyone of claim **5** to **10,** wherein udenafil and one of alfuzosin and oxybutynin are to be administrated orally.
